(19) Europäisches Patentamt
European Patent Office
Office européen des brevets

(11) Veröffentlichungsnummer: **0 533 014 A1**

(12) **EUROPÄISCHE PATENTANMELDUNG**

(21) Anmeldenummer: **92115274.0**

(22) Anmeldetag: **07.09.92**

(51) Int. Cl.[5]: **C07D 401/12**, C07D 401/14, A61K 31/445

(30) Priorität: **20.09.91 DE 4131346**

(43) Veröffentlichungstag der Anmeldung: **24.03.93 Patentblatt 93/12**

(84) Benannte Vertragsstaaten: **AT BE CH DE DK ES FR GB GR IE IT LI LU MC NL PT SE**

(71) Anmelder: **BAYER AG**

**W-5090 Leverkusen 1 Bayerwerk(DE)**

(72) Erfinder: **Niewöhner, Ulrich, Dr.**
**Gartenstrasse 3**
**W-5632 Wermelskirchen 3(DE)**
Erfinder: **Goldmann, Siegfried, Dr.**
**Am Osterholz 91**
**W-5600 Wuppertal 11(DE)**
Erfinder: **Müller, Ulrich, Dr.**
**Claudiusweg 5**
**W-5600 Wuppertal 1(DE)**
Erfinder: **Knorr, Andreas, Dr.**
**Trillser Graben 10**
**W-4006 Erkrath 2(DE)**
Erfinder: **Perzborn, Elisabeth, Dr.**
**Am Tescher Busch 13**
**W-5600 Wuppertal 11(DE)**
Erfinder: **Schramm, Matthias, Dr.**
**Humperdinck-Strasse 15**
**W-5090 Leverkusen(DE)**
Erfinder: **Beckermann, Bernhard, Dr.**
**Langemarckweg 59**
**W-5060 Bergisch Gladbach 2(DE)**

(54) **Indolsulfonamid substituierte Dihydropyridine, Verfahren zu ihrer Herstellung und ihre Verwendung in Arzneimitteln.**

(57) Indolsulfonamid substituierte Dihydropyridine können hergestellt werden durch Umsetzung von Dihydropyridincarbonsäuren mit Hydroxy substituierten Indolsulfonamiden, oder durch Umsetzung von amino- bzw. hydroxy substituierten Dihydropyridinen mit Indolsulfonamidcarbonsäuren. Die Indolsulfonamid substituierten Dihydropyridine können als Wirkstoffe in Arzneimitteln zur Behandlung von Herz-, Kreislauf- sowie thromboembolischen Erkrankungen eingesetzt werden.

EP 0 533 014 A1

Die Erfindung betrifft neue Indolsulfonamid substituierte Dihydropyridine, Verfahren zu ihrer Herstellung und ihre Verwendung in Arzneimitteln, insbesondere für Herz-, Kreislauf- und thromboembolische Erkrankungen.

Es ist bereits bekannt, daß 4-Pyridyl- und 4-Phenyl-1,4-dihydropyridin-3,5-dicarboxylate eine calciumantagonistische und hypotensive Wirkung besitzen [vgl. EP 265 947]. Außerdem werden in der DOS 36 05 566 Cycloalkano[1,2-b]indolsulfonamide beschrieben.

Die Erfindung betrifft neue Indolsulfonamid substituierte Dihydropyridine der allgemeinen Formel (I)

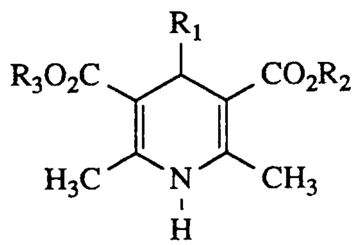

(I),

in welcher

| | |
|---|---|
| $R^1$ | für Aryl mit 6 bis 10 Kohlenstoffatomen oder für einen 5- bis 7-gliedrigen ungesättigten Heterocyclus mit bis zu 2 Heteroatomen aus der Reihe S, N oder O steht, die gegebenenfalls bis zu 3-fach gleich oder verschieden durch Halogen, Nitro, Trifluormethyl, Trifluormethoxy, Cyano, Difluormethoxy, geradkettiges oder verzweigtes Alkyl, Alkoxy oder Alkylthio mit jeweils bis zu 8 Kohlenstoffatomen, Benzyl oder Phenoxy substituiert sind, |
| $R^2$ | für einen Rest der Formel |

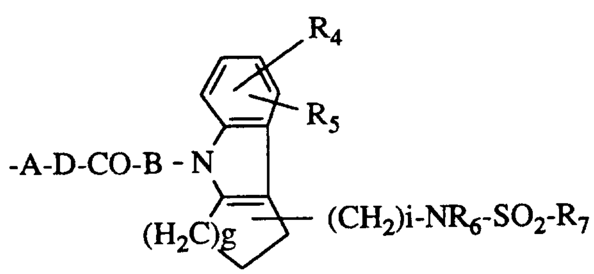

steht,

| | |
|---|---|
| | worin |
| A und B | gleich oder verschieden sind und eine Gruppe der Formel $-(CH_2)_b-(CR^8R^9)_d-(CH_2)_e$ bedeuten, |
| | worin |
| b | eine Zahl 1, 2, 3, 4 oder 5 bedeutet, |
| d und e | gleich oder verschieden sind und eine Zahl 0, 1, 2, 3, 4 oder 5 bedeuten, |
| $R^8$ und $R^9$ | gleich oder verschieden sind und Wasserstoff oder geradkettiges oder verzweigtes Alkyl mit bis zu 6 Kohlenstoffatomen bedeuten, |
| D | ein Sauerstoffatom oder die -NH-Gruppe bedeutet, |
| g | eine Zahl 1 oder 2 bedeutet, |
| i | eine Zahl 0, 1, 2 oder 3 bedeutet, |
| $R^4$ und $R^5$ | gleich oder verschieden sind und Wasserstoff, Aryl mit 6 bis 10 Kohlenstoffatomen, Nitro, Cyano, Halogen, Trifluormethyl, Trifluormethoxy, Carboxy, Hydroxy oder geradkettiges oder verzweigtes Alkyl, Alkoxy oder Alkoxycarbonyl mit jeweils bis zu 6 Kohlenstoffatomen bedeuten, |
| $R^6$ | Wasserstoff, geradkettiges oder verzweigtes Alkyl mit bis zu 8 Koh- |

lenstoffatomen oder Phenyl bedeutet,

$R^7$ Aryl mit 6 bis 10 Kohlenstoffatomen oder einen 5- bis 7-gliedrigen ungesättigten Heterocyclus mit bis zu 2 Heteroatomen aus der Reihe S, H oder O bedeutet, die gegebenenfalls bis zu 3-fach gleich oder verschieden durch Halogen, Cyano, Nitro, Trifluormethyl, Trifluormethoxy, Trifluormethylthio, Hydroxy, Carboxy, Phenyl, Phenoxy, Benzyloxy, Benzylthio, geradkettiges oder verzweigtes Alkoxy, Alkyl, Carboxyalkyl, Alkoxycarbonyl oder Alkoxycarbonylalkyl mit jeweils bis zu 8 Kohlenstoffatomen oder durch eine Gruppe der Formel $-NR^{10}R^{11}$ substituiert sind,

worin

$R^{10}$ und $R^{11}$ gleich oder verschieden sind und Wasserstoff, geradkettiges oder verzweigtes Alkyl mit bis zu 6 Kohlenstoffatomen, Phenyl oder Benzyl bedeuten,

$R^3$ für geradkettiges oder verzweigtes Alkyl oder Alkenyl mit jeweils bis zu 10 Kohlenstoffatomen steht, die gegebenenfalls bis zu 2-fach gleich oder verschieden durch Carboxy, geradkettiges oder verzweigtes Alkylthio, Alkoxy, Alkoxycarbonyl, Acyl oder Acyloxy mit jeweils bis zu 8 Kohlenstoffatomen, Phenyl, Phenoxy, Carboxy, Hydroxy oder durch die Gruppe $-NR^{10}R^{11}$ substituiert sind,

worin

$R^{10}$ und $R^{11}$ die oben angegebene Bedeutung haben,

oder für die Gruppe der Formel

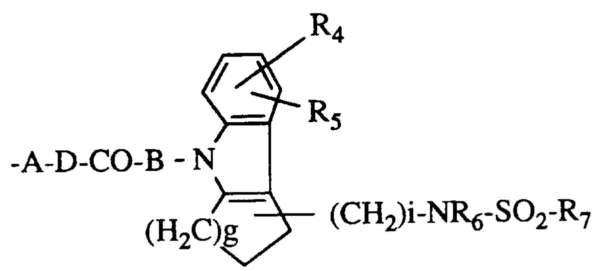

steht,

worin

A, B, D, g, i, $R^4$, $R^5$, $R^6$ und $R^7$ die oben angegebene Bedeutung haben,

gegebenenfalls in einer isomeren Form und deren Salze.

Die erfindungsgemäßen Verbindungen der allgemeinen Formel (I) haben mehrere asymmetrische Kohlenstoffatome und können daher in verschiedenen stereochemischen Formen auftreten, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Die erfindungsgemäßen Stoffe können auch als Salze vorliegen. Im Rahmen der Erfindung sind physiologisch unbedenkliche Salze bevorzugt.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Salze im Rahmen der vorliegenden Erfindung sind außerdem Salze der einwertigen Metalle wie Alkalimetalle und die Ammoniumsalze. Bevorzugt werden Natrium-, Kalium- und Ammoniumsalze.

Heterocyclus steht im allgemeinen für einen 5- bis 7-gliedrigen, bevorzugt 5- bis 6-gliedrigen ungesättigten Ring, der als Heteroatome bis zu 2 Sauerstoff-, Schwefel- und/oder Stickstoffatome enthalten kann.

Bevorzugt sind 5- und 6-gliedrige Ringe mit einem Sauerstoff-, Schwefel- und/oder bis zu 2 Stickstoffatome. Bevorzugt werden genannt: Thienyl, Furyl, Pyrrolyl, Pyyidyl oder Pyrimidyl.

Bevorzugt sind Verbindungen der allgemeinen Formel (I), in welcher

| | |
|---|---|
| $R^1$ | für Phenyl, Naphthyl, o-Pyridyl, m-Pyridyl, p-Pyridyl oder Thienyl steht, die gegebenenfalls bis zu 2-fach gleich oder verschieden durch Fluor, Chlor, Brom, Nitro, Trifluormethyl, Trifluormethoxy, geradkettiges oder verzweigtes Alkyl oder Alkoxy mit jeweils bis zu 6 Kohlenstoffatomen, Benzyl oder Phenoxy substituiert sind, |
| $R^2$ | für einen Rest der Formel 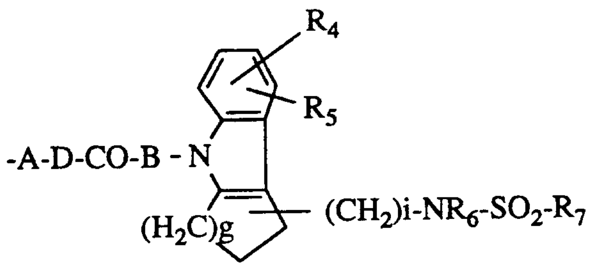 steht, worin |
| A und B | gleich oder verschieden sind und eine Gruppe der Formel $-(CH_2)_b-(CR^8R^9)_d-(CH_2)_e$ bedeuten, worin |
| b | eine Zahl 1, 2, 3 oder 4 bedeutet, |
| d und e | gleich oder verschieden sind und eine Zahl 0, 1, 2 oder 3 bedeuten, |
| $R^8$ und $R^9$ | gleich oder verschieden sind und Wasserstoff, geradkettiges oder verzweigtes Alkyl mit bis zu 4 Kohlenstoffatomen bedeuten, |
| D | ein Sauerstoffatom oder die -NH-Gruppe bedeutet, |
| g | eine Zahl 1 oder 2 bedeutet, |
| i | eine Zahl 0, 1 oder 2 bedeutet, |
| $R^4$ und $R^5$ | gleich oder verschieden sind und Wasserstoff, Phenyl, Nitro, Cyano, Fluor, Chlor, Brom, Trifluormethyl oder geradkettiges oder verzweigtes Alkyl oder Alkoxy mit jeweils bis zu 4 Kohlenstoffatomen bedeuten, |
| $R^6$ | Wasserstoff, geradkettiges oder verzweigtes Alkyl mit bis zu 6 Kohlenstoffatomen bedeutet, |
| $R^7$ | Phenyl, Naphthyl oder Thienyl bedeutet, die gegebenenfalls bis zu 2-fach gleich oder verschieden durch Fluor, Chlor, Trifluormethyl, Trifluormethoxy, Hydroxy, Carboxy oder durch geradkettiges oder verzweigtes Alkoxy, Alkyl oder Alkoxycarbonyl mit jeweils bis zu 6 Kohlenstoffatomen oder durch eine Gruppe der Formel $-NR^{10}R^{11}$ substituiert sind, worin |
| $R^{10}$ und $R^{11}$ | gleich oder verschieden sind und Wasserstoff, geradkettiges oder verzweigtes Alkyl mit bis zu 4 Kohlenstoffatomen, Phenyl oder Benzyl bedeuten, |
| $R^3$ | für geradkettiges oder verzweigtes Alkyl mit bis zu 8 Kohlenstoffatomen steht, das gegebenenfalls durch geradkettiges oder verzweigtes Alkoxy, Alkylthio, Alkoxycarbonyl, Acyl oder Acyloxy mit jeweils bis zu 6 Kohlenstoffatomen, Phenyl, Phenoxy, Carboxy oder Hydroxy oder durch die Gruppe $-NR^{10}R^{11}$ substituiert ist, worin |
| $R^{10}$ und $R^{11}$ | die oben angegebene Bedeutung haben, oder für die Gruppe der Formel |

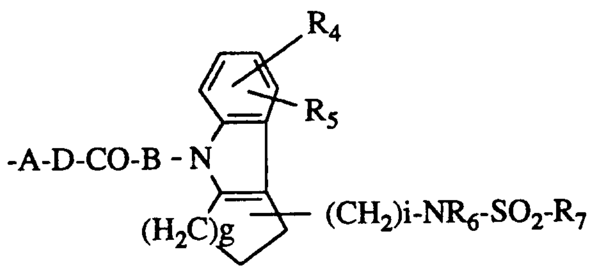

steht,
worin

A, B, D, g, i, $R^4$, $R^5$, $R^6$ und $R^7$ die oben angegebene Bedeutung haben,

gegebenenfalls in einer isomeren Form und deren Salze.

Besonders bevorzugt sind Verbindungen der allgemeinen Formel (I),
in welcher

$R^1$ für Phenyl, Naphthyl, m-Pyridyl oder Thienyl steht, die gegebenenfalls bis zu zweifach gleich oder verschieden durch Fluor, Chlor, Nitro, Trifluormethyl, Trifluormethoxy, geradkettiges oder verzweigtes Alkyl oder Alkoxy mit jeweils bis zu 4 Kohlenstoffatomen, Benzyl oder Phenoxy substituiert sind,

$R^2$ für einen Rest der Formel

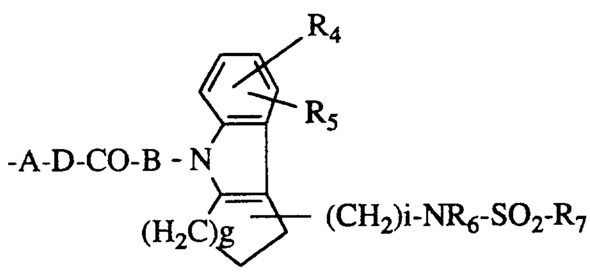

steht,
worin

A und B gleich oder verschieden sind und eine Gruppe der Formel $-(CH_2)_b-(CR^8R^9)_d-(CH_2)_e$ bedeuten,
worin

b eine Zahl 1, 2 oder 3 bedeutet,

d und e gleich oder verschieden sind und eine Zahl 0, 1 oder 2 bedeuten,

$R^8$ und $R^9$ gleich oder verschieden sind und Wasserstoff, Methyl oder Ethyl bedeuten,

D ein Sauerstoffatom oder die -NH-Gruppe bedeutet,

g eine Zahl 1 oder 2 bedeutet,

i eine Zahl 0 oder 1 bedeutet,

$R^4$ und $R^5$ gleich oder verschieden sind und Wasserstoff, Phenyl, Nitro, Fluor, Chlor, Trifluormethyl, Methyl oder Methoxy bedeuten,

$R^6$ Wasserstoff, geradkettiges oder verzweigtes Alkyl mit bis zu 4 Kohlenstoffatomen bedeutet,

$R^7$ Phenyl, Naphthyl oder Thienyl bedeutet, die gegebenenfalls durch Fluor, Chlor, Trifluormethyl, Trifluormethoxy oder durch geradkettiges oder verzweigtes Alkoxy oder Alkyl mit jeweils bis zu 4 Kohlenstoffatomen substituiert sind,

$R^3$ für geradkettiges oder verzweigtes Alkyl mit bis zu 6 Kohlenstoffatomen steht, das gegebenenfalls durch geradkettiges oder verzweigtes Alkoxy, Alkylthio, Alkoxycarbonyl oder Acyl mit jeweils bis zu 4

Kohlenstoffatomen, Phenyl oder Phenoxy substituiert ist, oder für die Gruppe der Formel

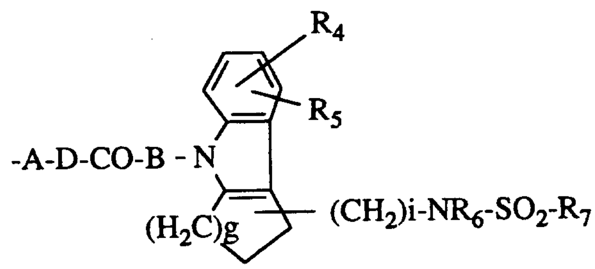

steht,
worin

A, B, D, g, i, $R^4$, $R^5$, $R^6$ und $R^7$ die oben angegebene Bedeutung haben,

gegebenenfalls in einer isomeren Form und deren Salze.

Ganz besonders bevorzugt sind Verbindungen der allgemeinen Formel (I) in welcher

$R^1$ für Phenyl oder Pyridyl steht, die gegebenenfalls durch Fluor, Chlor, Nitro oder Trifluormethyl substituiert sind,

$R^2$ für einen Rest der Formel

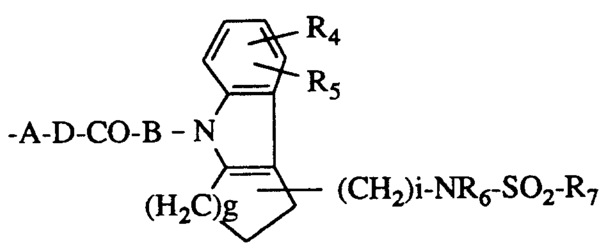

steht,
worin

A - eine Gruppe der Formel $-(CH_2)_b-(CR^8R^9)_d-(CH_2)_e$ bedeutet, wobei

b - fur eine Zahl 1, 2 oder 3 steht,

d, e - gleich oder verschieden sind und für eine Zahl 0, 1 oder 2 stehen

$R^8$, $R^9$ - gleich oder verschieden sind und für Wasserstoff oder Methyl stehen

D - ein Sauerstoffatom oder eine NH-Gruppe bedeutet

g - die Zahl 2 bedeutet

i - die Zahl 0 bedeutet

$R^4$ und $R^5$ - Wasserstoff bedeuten

$R^6$ - Wasserstoff bedeutet

$R^7$ - Phenyl bedeutet, das gegebenenfalls durch Fluor oder Chlor substituiert ist,

$R^3$ für geradkettiges oder verzweigtes Alkyl mit bis zu 6 Kohlenstoffatomen steht, oder für eine Gruppe der Formel

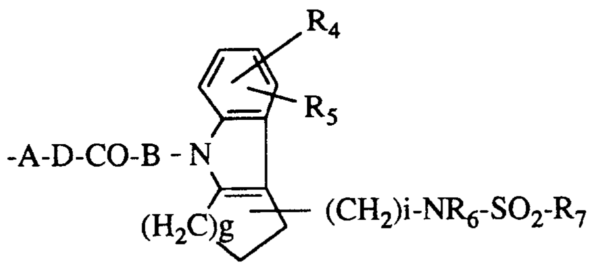

steht,
worin
A, B, D, g, i, $R^4$, $R^5$, $R^6$ und $R^7$ die oben angegebene Bedeutung haben
gegebenenfalls in einer isomeren Form und deren Salze.

Außerdem wurden Verfahren zur Herstellung der erfindungsgemäßen Verbindungen der allgemeinen Formel (I) gefunden, dadurch gekennzeichnet, daß man

[A] Dihydropyridincarbonsäuren der allgemeinen Formel (II)

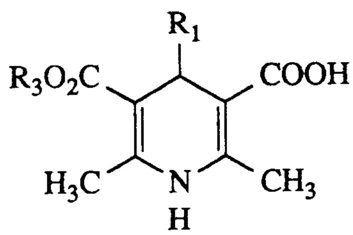

(II),

in welcher
$R^1$ und $R^3$ die oben angegebene Bedeutung haben
mit Indolsulfonamiden der allgemeinen Formel (III)

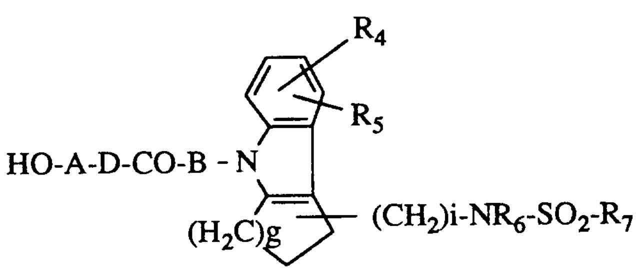

(III),

in welcher
A, D, B, g, i, $R^4$, $R^5$, $R^6$ und $R^7$ die oben angegebene Bedeutung haben,
in inerten Lösemitteln, gegebenenfalls in Anwesenheit einer Base und/oder eines Hilfsstoffes, und gegebenenfalls unter Aktivierung der Carbonsäure verestert,
oder daß man

[B] Dihydropyridine der allgemeinen Formel (IV)

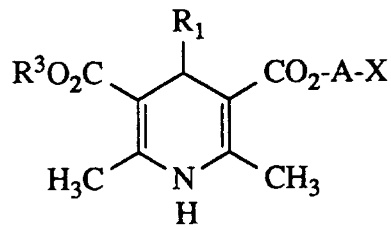

(IV),

worin

R¹, R³ und A — die oben angegebene Bedeutung haben und

X — entweder für eine -OH-Gruppe steht, wenn D ein Sauerstoffatom bedeutet, oder für eine $-NH_2$-Gruppe steht, wenn D eine -NH-Gruppe bedeutet,

mit Indolsulfonamidsäuren der allgemeinen Formel (V),

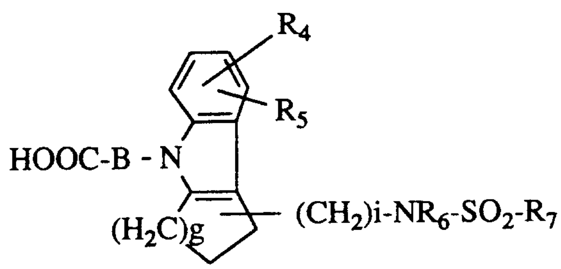

(V),

in welcher

B, g, i, $R^4$, $R^5$, $R^6$ und $R^7$ die oben angegebene Bedeutung haben, in inerten Lösemitteln, gegebenenfalls in Anwesenheit eines Hilfstoffes und gegebenenfalls unter Aktiviervng der Carbonsäure kondensiert, und im Fall der reinen Enantiomeren zunächst auf der Stufe der Säuren der allgemeinen Formel (II) eine Enantiomerentrennung nach üblicher Methode durchführt und die entsprechenden enantiomerenreinen Säuren mit den ebenfalls nach einer Diastereomerentrennung im Verlauf der Synthese anfallenden enantiomerenreinen Verbindungen der allgemeinen Formel (III) oder nach Überführung der enantiomerenreinen Säuren (II) in die enantiomerenreinen Verbindungen (IV) mit enantiomerenreinen Verbindungen der allgemeinen Formel (V) umsetzt.

Die erfindungsgemäßen Verfahren können durch folgende Formelschemen beispielhaft erläutert werden:

[B]

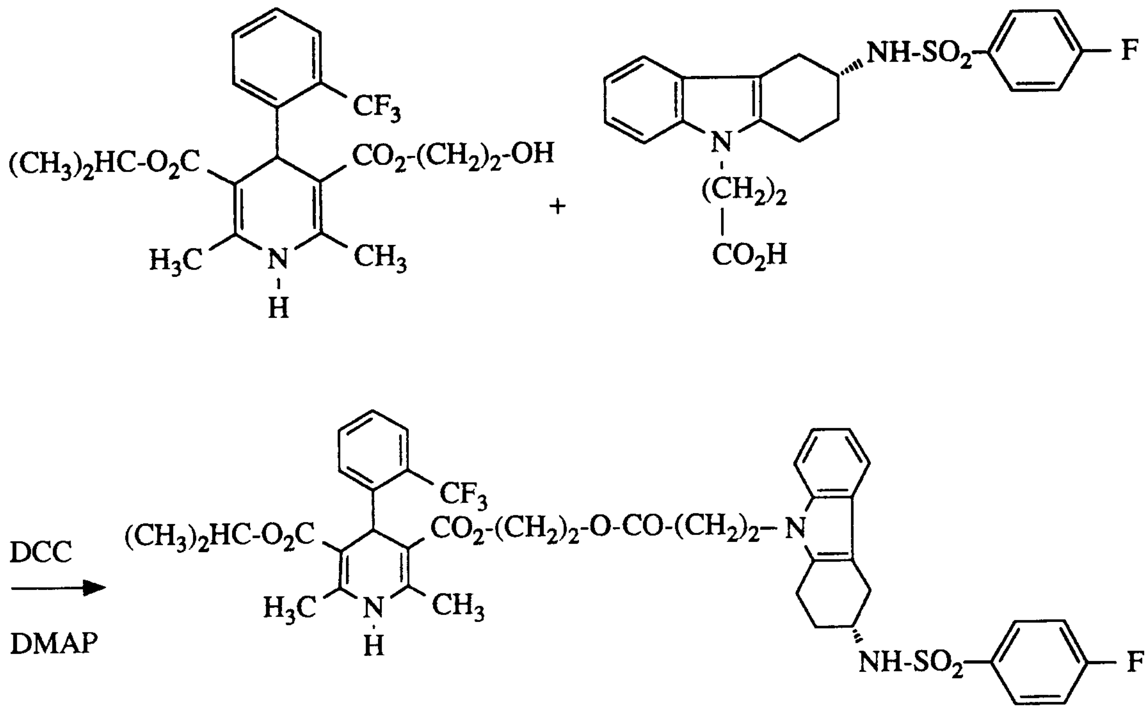


[A]

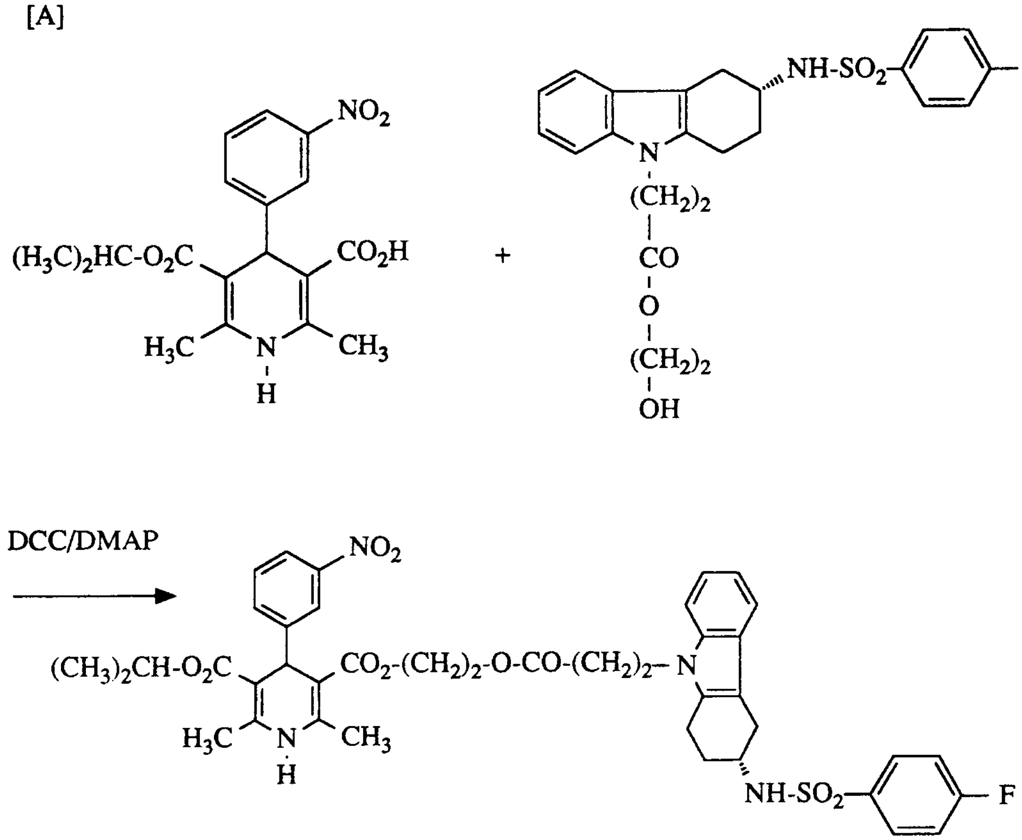

[A]

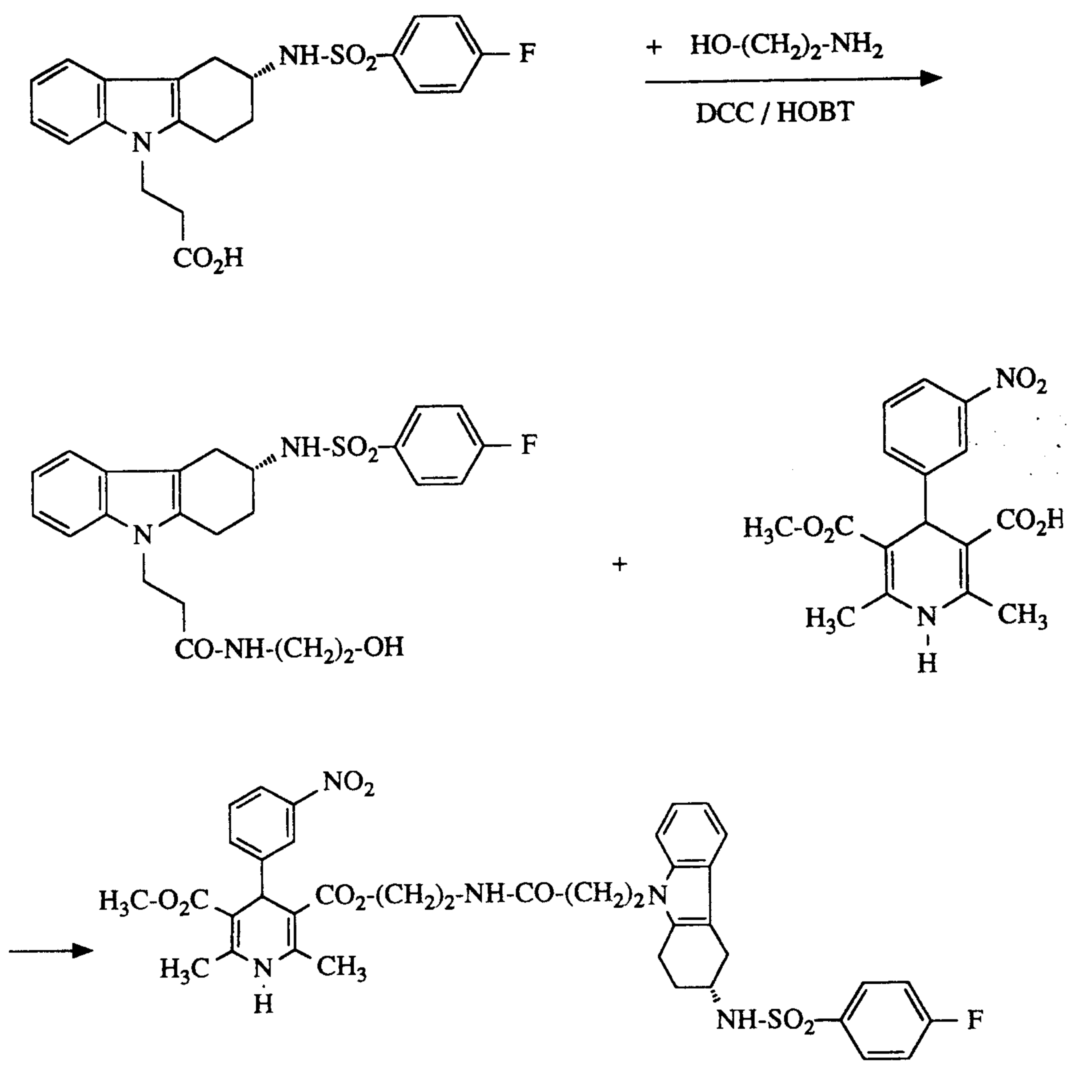

[B]

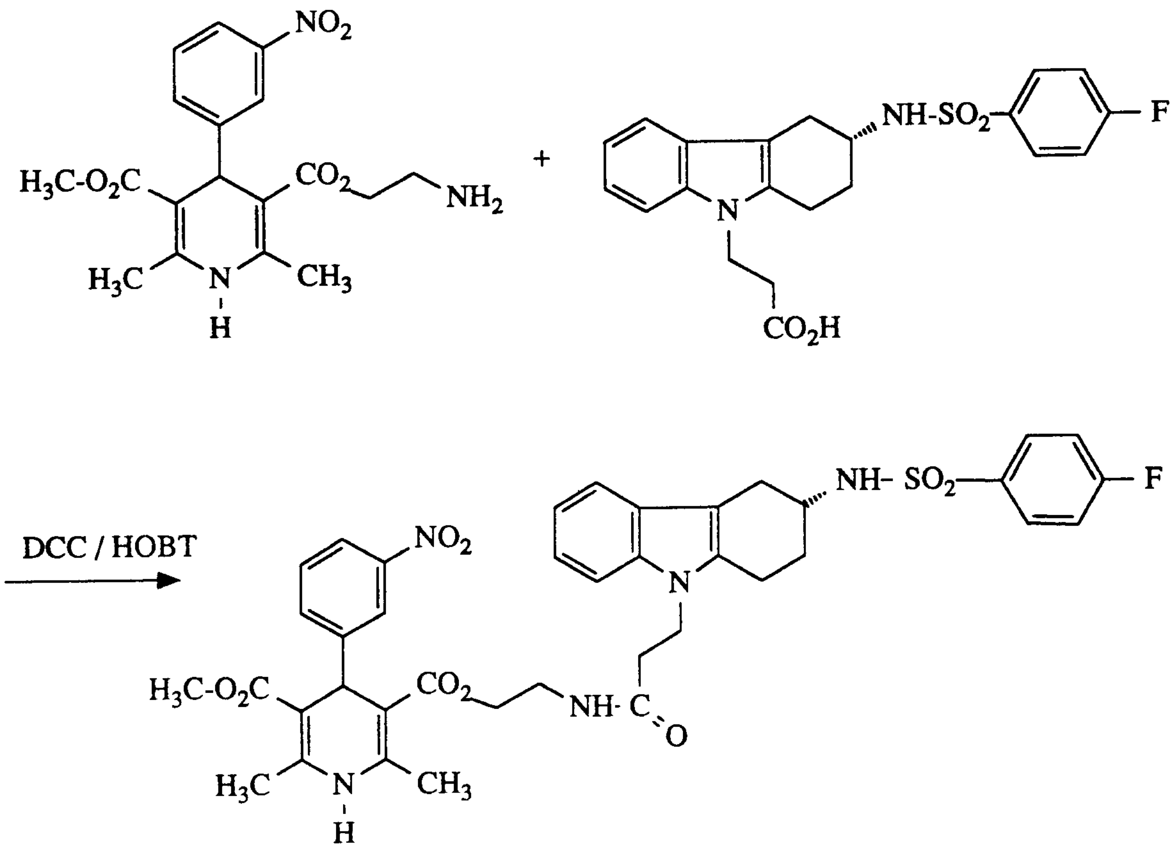


Lösemittel für die erfindungsgemäßen Verfahren [A] und [B] können hier inerte organische Lösemittel sein, die sich unter den Reaktionsbedingungen nicht verändern. Hierzu gehören bevorzugt Ether wie beispielsweise Diethylether, Dioxan, Tetrahydrofuran, Glykolmono- oder dimethylether, Halogenkohlenwasserstoffe wie Di-, Tri- oder Tetrachlormethan, Dichlorethylen, Trichlorethylen, Essigester, Toluol, Acetonitril, Hexamethylphosphorsäuretriamid und Aceton. Selbstverständlich ist es möglich, Gemische der Lösemittel einzusetzen. Bevorzugt ist Tetrahydrofüran.

Als Hilfsstoffe werden bevorzugt Kondensationsmittel eingesetzt die auch Basen sein können. Bevorzugt werden hier die üblichen Kondensationsmittel wie Carbodiimide z.B. N,N'-Diethyl-, N,N'-Dipropyl-, N,N'-Diisopropyl-, N,N'-Dicyclohexylcarbodiimid, N-(3-Dimethylaminoisopropyl)-N'-ethylcarbodiimid-Hydrochlorid, oder Carbonylverbindungen wie Carbonyldiimidazol, oder 1,2-Oxazoliumverbindungen wie 2-Ethyl-5-phenyl-1,2-oxazolium-3-sulfonat oder 2-tert.Butyl-5-methylisoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Benzotriazolyloxy-tris(dimethylamino)phosphonium-haxafluorophosphonat. Bevorzugt sind N,N'-Dicyclohexylcarbodiimid und Carbonyldiimidazol.

Als Basen eignen sich im allgemeinen Alkalicarbonate wie beispielsweise Natrium- oder Kaliumcarbonat, oder organische Basen wie Trialkylamine, z.B. Triethylamin, N-Ethylmorpholin, N-Methylpiperidin oder Diisopropylethylamin, oder Dimethylaminopyridin, 1,8-Diazabicyclo[5.4.0]undec-7-en (DBU) oder 1,5-Diazabicyclo[4.3.0]non-5-en (DBN). Bevorzugt ist Dimethylaminopyridin.

Die Base wird im allgemeinen in einer Menge von 0,01 mol bis 1 mol, bevorzugt von 0,05 mol bis 0,1 mol, jeweils bezogen auf 1 mol der Verbindungen der allgemeinen Formel (II) oder (IV) eingesetzt.

Die Hilfsstoffe werden im allgemeinen in einer Menge von 1 mol bis 3 mol, bevorzugt von 1 mol bis 1,5 mol, jeweils bezogen auf 1 mol der Verbindungen der allgemeinen Formeln (II) und (IV) eingesetzt.

Die Reaktionstemperatur für die Verfahren [A] und [B] können in einem größeren Bereich variiert werden. Im allgemeinen arbeitet man in einem Bereich von -20 °C bis 200 °C, bevorzugt von 0 °C bis 25 °C.

Die Verfahren können bei Normaldruck, erhöhtem oder erniedrigtem Druck (beispielsweise von 0,5 bis 5 bar), vorzugsweise bei Normaldruck durchgeführt werden.

Bei der Durchführung der erfindungsgemäßen Verfahren ist das Verhältnis der an der Reaktion beteiligten Stoffe beliebig. Im allgemeinen arbeitet man jedoch bei molaren Mengen der Reaktanden.

Zur Aktivierung der Carbonsäure eignen sich die üblichen Reagenzien wie anorganische Halogenide, beispielsweise Thionylchlorid, Phosphortrichlorid oder Phosphorpentachlorid, oder Carbonyldiimidazol, Carbodiimide wie Cyclohexylcarbodiimid oder 1-Cyclohexyl-3-[2-(N-methylmorpholino)ethyl]-carbodiimid-p-toluolsulfonat oder N-Hydroxyphthalimid oder N-Hydroxy-benztriazol.

Enantiomerenreine Formen erhält man z.B. außerdem, indem man Diastereomerengemische der Verbindungen der allgemeinen Formel (I), in welcher $R^2$ oder $R^3$ für einen optischen Esterrest steht, nach üblicher Methode trennt, anschließend die enantiomerenreinen Carbonsäuren herstellt und dann gegebenenfalls durch Veresterung mit entsprechenden Alkoholen in die enantiomerenreinen Dihydropyridine überführt.

Geeignet als chirale Esterreste sind alle Ester enantiomerenreiner Alkohole wie beispielsweise 2-Butanol, 1-Phenylethanol, Milchsäure, Milchsäureester, Mandelsäure, Mandelsäureester, 2-Aminoalkohole, Zuckerderivate und viele andere enantiomerenreine Alkohole mehr.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die Veresterung der enantiomerenreinen Dihydropyridine erfolgt bevorzugt in Ethem wie Diethylether oder Tetrahydrofuran, Dimethylformamid, Methylenchlorid, Chloroform, Acetonitril oder Toluol.

Bevorzugt erfolgt die Trennung der Enantiomere der Verbindungen der allgemeinen Formel (II) durch Säulenchromatographie nach üblicher Methode, wobei die Säulen mit chiralen Trägermaterialien gefüllt sind.

Die vorstehenden Herstellungsverfahren sind lediglich zur Verdeutlichung angegeben. Die Herstellung der Verbindungen der Formel (I) ist nicht auf diese Verfahren beschränt, sondern jede Modifikation dieser Verfahren ist in gleicher Weise für die Herstellung der erfindungsgemäßen Verbindungen anwendbar.

Die Verbindungen der allgemeinen Formel (IV) sind größtenteils bekannt [vgl. US 44 19 518] oder können nach den in der Dihydropyridinchemie üblichen Methoden, beispielsweise unter Aktivierung der jeweiligen Carbonsäuren mit einem der oben aufgeführten Hilfsstoffe, vorzugsweise Dicyclohexylcarbodiimid oder Carbonyldiimidazol und nachfolgender Umsetzung mit Verbindungen der allgemeinen Formel (VI)

HO-A-X (VI),

in welcher

A und X die oben angegebene Bedeutung haben,

hergestellt werden.

Die Verbindungen der allgemeinen Formel (V) sind bekannt [vgl. DOS 36 05 566].

Die Verbindungen der allgemeinen Formel (II) sind im Fall, daß $R^3$ für den Rest der Formel

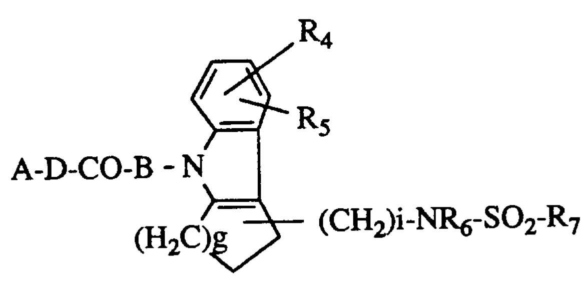

steht, worin

A, B, D, $R^4$, $R^5$, $R^6$, $R^7$, g und i die oben angegebene Bedeutung haben,

neu und können nach der unter [A] angegebenen Methode hergestellt werden.

Die Verbindungen der allgemeinen Formel (III) sind neu und können hergestellt werden, indem man zunächst die Carbonsäurefunktion in den Verbindungen der allgemeinen Formel (V), gegebenenfalls in Anwesenheit einer der oben aufgeführten Basen und/oder Hilfsstoffe, bevorzugt mit Dicyclohexylcarbodiimid und Dimethylaminopyridin, aktiviert, anschließend mit Verbindungen der allgemeinen Formel (VII)

EO-A-OH (VII),

in welcher

A die oben angegebene Bedeutung hat,

und

E für eien typische Hydroxyschutzgruppe, bevorzugt Benzyl steht,

in inerten Lösemitteln, in Anwesenheit einer Base umsetzt, und in einem letzten Schritt diese nach üblicher Methode abspaltet.

Als Lösemittel eignen sich die oben aufgeführten Lösemittel, vorzugsweise Tetrahydrofuran und Methylenchlorid.

Als Basen eignen sich Alkali- und Erdalkalihydroxide, wie Natrium- oder Kaliumhydroxid. Bevorzugt ist Natriumhydroxid.

Die Base wird in einer Menge von 0,01 mol bis 1 mol, bevorzugt von 0,05 mol bis 0,15 mol, bezogen auf 1 mol der Verbindung der allgemeinen Formel (V) eingesetzt.

Die Reaktionstemperatur kann in einem größeren Bereich variiert werden. Im allgemeinen arbeitet man in einem Bereich von -20°C bis 200°C, bevorzugt von 0°C bis 25°C.

Die Verfahren können bei Normaldruck, erhöhtem oder erniedrigtem Druck (beispielsweise von 0,5 bis 5 bar), vorzugsweise bei Normaldruck durchgeführt werden.

Hydroxyschutzgruppe im Rahmen der oben angegebenen Definition steht im allgemeinen für eine Schutzgruppe aus der Reihe: Trimethylsilyl, Triethylsilyl, Triisopropylsilyl, tert.Butyl-dimethylsilyl, Triphenylsilyl oder Benzyl. Bevorzugt ist Trimethylsilyl, tert.Butyl-dimethylsilyl oder Benzyl.

Die Abspaltung der Schutzgruppen aus den entsprechenden Ethern erfolgt nach üblicher Methode, beispielsweise durch hydrogenolytische Spaltung der Benzylether in den oben aufgeführten inerten Lösemitteln in Anwesenheit eines Katalysators mit Wasserstoff-Gas [vgl. außerdem Th. Green: "Protective Groups in Organic Synthesis", J. Wiley & Sons, 1981, New York].

Die Verbindungen der allgemeinen Formel (VI) und (VII) sind an sich bekannt oder können nach üblicher Methode hergestellt werden [vgl. Beilstein 1 467; 1, 2, 519].

Die erfindungsgemäßen Verbindungen der allgemeinen Formel (I) zeigen ein nicht vorhersehbares, wertvolles pharmakologisches Wirkspektrum auf. Sie beeinflussen zum einen die Kontraktionskraft des Herzens, den Tonus der glatten Muskulatur sowie den Elektrolyt- und Flüssigkeitshaushalt. Außerdem weisen sie eine thrombozyten-aggregationshemmende und Thromboxan-$A_2$-antagonistische Wirkung auf. Sie können daher in Arzneimitteln zur Behandlung des pathologisch veränderten Blutdrucks und der Herzinsuffizienz, sowie als Koronartherapeutika eingesetzt werden. Sie können zur Behandlung von thromboembolischen Erkrankungen und Ischämien wie Myokardinfarkt, Hirnschlag, transitorisch und ischämische Attacken, Angina pectoris, periphere Durchblutungsstörungen, Verhinderung von Restenosen wie nach Thrombolysetherapie, percutan transluminalen Angioplastien (PTA), percutanen transluminalen Koronarangioplastien (PTCA), Bypass sowie zur Behandlung von Arteriosklerose, Asthma und Allergien eingesetzt werden.

Die Substanzen wurden an der wachen Ratte auf ihre blutdrucksenkende Wirkung hin untersucht. An wachen Ratten mit genetisch bedingtem Hochdruck ("spontan hypertone Ratten" des Okamoto-Stammes) wird der arterielle Blutdruck mit der "Schwanz-Manchette" in definierten Zeitabständen nach Substanzgabe unblutig gemessen. Die zu prüfenden Substanzen werden aufgeschwemmt in einer Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung.

| Es ergaben sich folgende Wirkungen: SH-Ratte: Blutdrucksenkung > 15 mm | |
|---|---|
| Bsp.-Nr. | mg/kg |
| 2 | 100 |
| 10 | 100 |
| 15 | 100 |
| 31 | 100 |

Bei diesen Ratten wurde auch die Thrombozytenaggregationshemmung ex vivo (Ratte) nach folgender Methode untersucht.

Die Prüfsubstanzen bzw. Lösemittel werden mittels Schlundsonde Ratten appliziert. Nach 60 Minuten wird den Tieren unter Ethernarkose über die Bauchaorta Blut entnommen. Das Blut wird in 3,8%iger Citrat-Lösung (9 +1) aufgenommen. Anschließend wird durch Zentrifugation bei 150 g für 20 Minuten plättchenreiches Plasma (PRP) gewonnen. 3 Teile PRP werden mit 1 Teil 0,9% NaCl verdünnt und bei 37°C 5

Minuten vorinkubiert.
Die Plättchenaggregation wird nach der turbidometrischen Methode [vgl. Born, G.V.R.; J. Physiol. 162, 67,1962] im Aggregometer bei 37°C bestimmt. Hierzu wird PRP mit Kollagen, einem aggregationsauslösenden Agens, versetzt. Die Prüfung erfolgt jeweils bei Schwellenkonzentrationen von Kollagen, die beim Kontrollansatz maximale Aggregation bewirken. Die Veränderung der optischen Dichte der aggregierenden Probe wird erfaßt und der maximale Ausschlag bestimmt. Hierzu wird die prozentuale Hemmung gegenüber der Kontrolle errechnet. Es ergab sich eine Hemmung von mehr als 50%.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös.

Im allgemeinen hat es sich als vorteilhaft erwiesen, bei intravenöser Applikation Mengen von etwa 0,01 bis 10 mg/kg, vorzugsweise etwa 0,1 bis 3 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Laufmittel

a) Toluol : Essigester = 4:1
b) Dichlormethan : Methanol = 20:1
c) Toluol : Essigester = 1:1
d) Toluol : Essigester = 1:2
e) Toluol : Essigester = 2:1
f) Dichlormethan : Petrolether = 3:1
g) Petrolether: Essigester = 1:1
h) Toluol : Essigester = 1:5
i) Petrolether : Essigester = 2:3
j) Cyclohexan : Essigester = 1:1
k) Toluol : Aceton = 5:1

Ausgangsverbindungen

Beispiel I

4-(3-Chlorphenyl)-5-(2-hydroxyethoxycarbonyl)-2,6-dimethyl-1,4-dihydropyridin-3-carbonsäureethylester

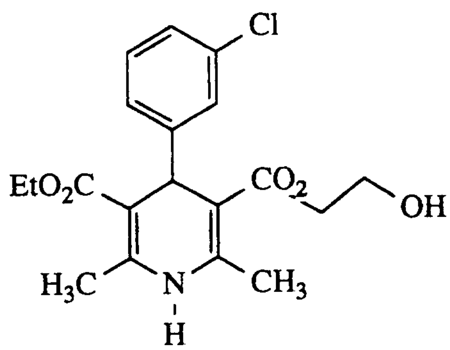

3,4 g (10 mmol) 4-(3-Chlorphenyl)-3-(ethoxycarbonyl)-2,6-dimethyl-1,4-dihydropyridin-5-carbonsäure und 2 g (10 mmol) Carbonyldimidazol werden in 50 ml abs. THF über Nacht bei Raumtemperatur gerührt. Es wird eingedampft, der Rückstand in 50 ml Essigsäureethylester aufgenommen und 2 mal mit je 50 ml Wasser gewaschen. Nach Trocknen über $Na_2SO_4$ wird das Lösemittel abdestilliert und der Rückstand (3,55 g) in 10 ml abs. THF gelöst. Nach Zugabe von 100 ml Ethylenglykol und 100 mg NaH wird 3 h bei Raumtemperatur gerührt, mit 100 ml Essigester versetzt und 2 mal mit je 100 ml Wasser ausgeschüttelt. Nach Trocknen über Natriumsulfat wird eingedampft. Der Rückstand ist im DC einheitlich und wird direkt weiterverarbeitet.
Ausbeute: 2,95 g (83% der Theorie)

Beispiel II

(3R)-3-(4-Fluorphenylsulfonylamido)-1,2,3,4-tetrahydrocarbazol-9-propansäure-benzylethylenglykolester

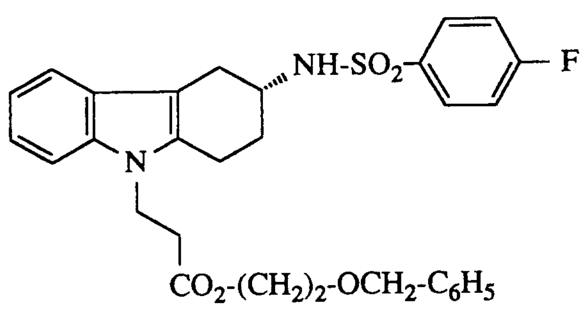

Zu einer Lösung von 41,6 g (0,1 mol) (3R)-3-(4-Fluorphenylsulfonamido)-1,2,3,4-tetrahydrocarbazol-9-propansäure in 500 ml Methylenchlorid werden bei 0 ° C nacheinander 16,7 g (0,11 mol) Monobenzylethylenglykol, 22,7 g (0,11 mol) Dicyclohexylcarbodiimid und 1 g DMAP gegeben. Es wird 30 Minuten bei 0 ° C, dann 2 h bei Raumtemperatur gerührt, filtriert und 2 mal mit 1 n NaOH, 1 mal mit 1 m HCl gewaschen. Nach Trocknen über $Na_2SO_4$ wird das Methylenchlorid abdestilliert und der Rückstand über eine kurze Säule durch Flashchromatographie gereinigt (Eluens: Toluol / Aceton 20:4).
Ausbeute: 51,7 g (94% der Theorie)

Beispiel III

(3R)-3-(4-Fluorphenylsulfonamido)-1,2,3,4-tetrahydrocarbazol-9-propansäureethylenglykolester

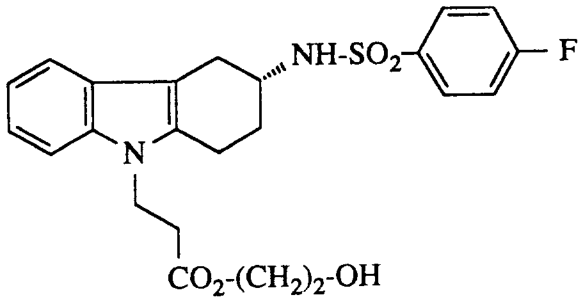

55 g (0,1 mol) der Verbindung aus Beispiel II werden in 800 ml abs. THF mit 15 g 10%igem Palladium/C bei 50 bar und 20 °C 12 h hydriert. Der Katalysator wird abfiltriert, das Lösemittel abdestilliert und der Rückstand durch Flashchromatographie gereinigt (Eluens: Toluol / Aceton 10:1 bis 5:1).
Ausbeute: 29,5 g (64,1% der Theorie)
$R_f$ = 0,48 (Toluol / Aceton 1:1)

Beispiel IV

(3R)-3-(4-Fluorphenylsulfonylamido)-1,2,3,4-tetrahydrocarbazol-9-propionsäureethylenglykolamid

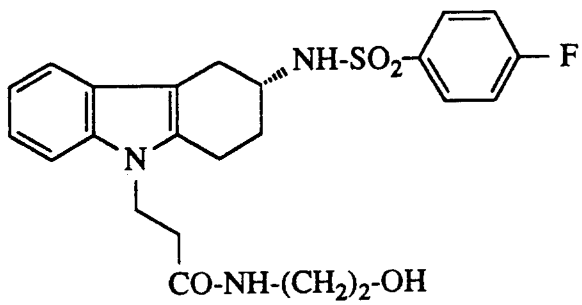

41,6 g (0,1 mol) (3R)-3-(4-Fluorphenylsulfonamido)-1,2,3,4-tetrahydrocarbazol-9-propansäure und 13,5 g (0,1 mol) 1-Hydroxybenzotriazol werden in 500 ml abs. $CH_2Cl_2$ gelöst und bei 0 °C mit 22,7 g (0,11 mol) Dicyclohexylcarbodiimid versetzt. Es wird 1 h bei 0 °C und 1 h bei 20 °C gerührt. Danach werden 6,1 g (0,1 mol) 2-Aminoethanol in 20 ml abs. $CH_2Cl_2$ zugetropft und 3 h bei 20 °C gerührt. Der Niederschlag wird abfiltriert, die $CH_2Cl_2$-Phase mit 1 n NaOH, 1 n HCl und gesättigter NaCl-Lösung gewaschen. Nach Trocknen über $Na_2SO_4$ wird das Lösemittel abdestilliert und der Rückstand durch Flashchromatographie gereinigt (Eluens: Toluol / Aceton 4:1 bis 2:1).
Ausbeute: 33,5 g (73% der Theorie)
$R_f$ = 0,36 (Toluol / Aceton 1:1)

Beispiel V

4-(3-Trifluormethylphenyl)-3-methoxycarbonyl-5-(2-phthalimidomethoxycarbnyl)-2,6-dimethyl-1,4-dihydropyridin

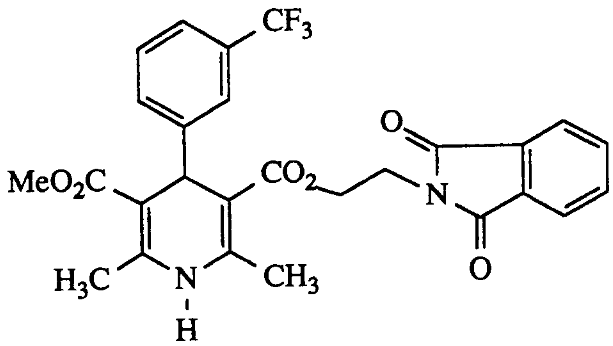

3,6 g (10 mmol) 4-(3-Trifluormethylphenyl)-3-methoxycarbonyl-2,6-dimethyl-1,4-dihydropyridin-5-carbonsäure und 2 g (10 mmol) Carbonyldiimidazol werden in 50 ml abs. THF über Nacht bei Raumtemperatur geruhrt. Es wird eingedampft, der Rückstand in 50 ml Essigsäureethylester aufgenommen und 2 mal mit je 50 ml Wasser gewaschen. Nach Trocknen über $Na_2SO_4$ wird das Lösemittel abdestilliert und der Rückstand (3,7 g) in 100 ml abs. THF gelöst Nach Zugabe von 1,91 g (10 mmol) N-(2-Hydroxyethyl)-phthalimid und 300 mg NaH wird 3 h bei Raumtemperatur gerührt. Es wird eingedampft und der Rückstand über Kieselgel mit Toluol/Aceton 10:1 als Eluens chromatographiert

Rf = 0,58 (Tol/Ac 1:1)

Ausbeute: 3,14 g (59,5 % der Theorie)

Beispiel VI

4-(3-Trifluormethylphenyl)-3-methoxycarbonyl-5-(2-aminoethoxycarbonyl)-1,4-dihydropyridin

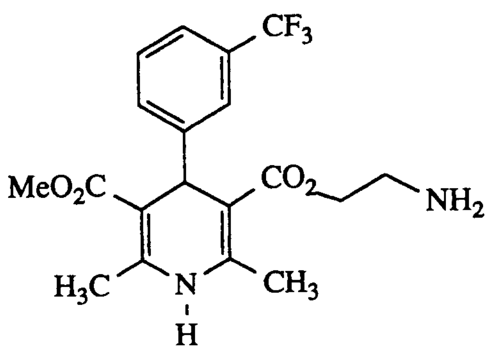

2,64 g (5 mmol) der Verbindung aus Beispiel V und 1,5 g Hydrazinhydrat werden in 50 ml Ethanol 1 h am Rückfluß gekocht. Es wird heiß abfiltiert, abgekühlt, nochmals filtriert und eingedampft. Der Rückstand wird über Kieselgel mit $CH_2Cl_2$/MeOH 10:1 als Eluens chromatographiert.

$R_f$ = 0,27 ($CH_2Cl_2$/MeOH 10:1)

Ausbeute: 1,27 g (63,8% der Theorie)

Herstellungsbeispiele

Methode B:

Allgemeine Arbeitsvorschrift zur Herstellung der Verbindungen der allgemeinen Formel (I), in welcher D für ein Sauerstoffatom steht, durch Veresterung verschiedener DHP-Ethylenglykolester der allgemeinen Formeln (IV) mit den Verbindungen der allgemeinen Formel (V).

7 mmol DHP-Ethylenglykolester und 2,91 g (7 mmol) (3R)-3-(4-Fluorphenylsulfonamido)-1,2,3,4-tetrahydrocarbazol-9-propionsäure werden in 20 mmol abs. THF gelöst. Bei 0°C werden 100 mg DMAP und 1,87 g (9,1 mmol) Dicyclohexylcarbodiimid zugegeben. Es wird 30 min bei 0°C, dann 3 h bei 20°C gerührt. Der Niederschlag wird abfiltriert, das THF abdestilliert und der Rückstand in 50 ml Essigsäureethylester aufgenommen. Es wird 2 mal mit gesättigter aq. $Na_2CO_3$-Lösung, 1 mal mit 1 n HCl und 1 x mit gesättiger NaCl-Lösung ausgeschüttelt, die organische Phase über $Na_2SO_4$ getrocknet und eingedampft. Der Rückstand wird durch Chromatographie gereinigt (Eluens: Toluol / Aceton-Gemische 25:1 bis 5:1).

Methode A:

Allgemeine Arbeitsvorschrift zur Herstellung der Verbindungen der allgemeinen Formel (I), in welcher D für ein Sauerstoffatom steht, durch Veresterung verschiedener DHP-Halbester der allgemeinen Formel (II) mit (3R)-3-(4-Fluorphenylsulfonamido)-1,2,3,4-tetrahydrocarbazol-9-propansäureethylenglykolester (Verbindung aus Beispiel III).

7 mmol DHP-Halbester und 3,22 g (7 mmol) der Verbindung aus Beispiel III werden in 20 ml abs. THF gelöst. Bei 0°C werden 100 mg DMAP und 1,87 g (9,1 mmol) Dicyclohexylcarbodiimid zugegeben. Es wird 30 min bei 0°C, dann 3 h bei 20°C gerührt. Der Niederschlag wird abfiltriert, das THF abdestilliert und der Rückstand in 50 ml Essigsäureethylester aufgenommen. Es wird 2 mal mit gesättigter aq. $Na_2CO_3$-Lösung, 1 mal mit 1 n HCl und 1 x mit gesättiger NaCl-Lösung ausgeschüttelt, die organische Phase über $Na_2SO_4$ getrocknet und eingedampft. Der Rückstand wird durch Chromatographie gereinigt (Eluens: Toluol / Aceton-Gemische 25:1 bis 5:1).

Die in Tabellen 1, 2 und 3 aufgeführten Verbindungen werden nach den oben angegebenen allgemeinen Herstellungsvorschriften hergestellt:

Tabelle 1:

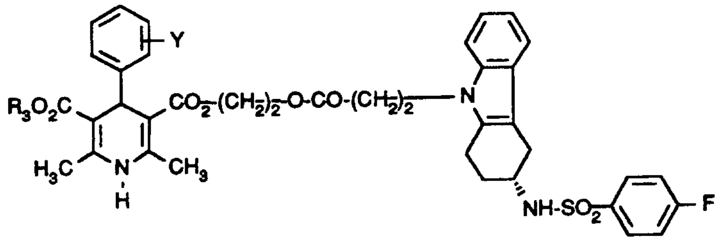

| Bsp.-Nr. | Y | $R^3$ | Ausbeute (% d.Th.) / $R_f$ (LM) | |
|---|---|---|---|---|
| 1 | o-$CF_3$ | -$CH(CH_3)_2$ | 61,5 | 0,28 (c) |
| 2 | m-$NO_2$ | -$C_2H_5$ | 66,4 | 0,39 (e) |
| 3 | m-$NO_2$ | -$CH(CH_3)_2$ | 86,1 | 0,15 (a) |
| 4*2 | m-$NO_2$ | -$CH(CH_3)_2$ | 89,3 | 0,15 (a) |
| 5*2 | m-$NO_2$ | -$CH(CH_3)_2$ | 77,0 | 0,15 (a) |
| 6 | m-$NO_2$ | -n-$C_5H_{11}$ | 64,3 | 0,68 (i) |
| 7 | m-$NO_2$ | -n-$C_6H_{13}$ | 59,8 | 0,48 (g) |
| 8 | m-$NO_2$ | 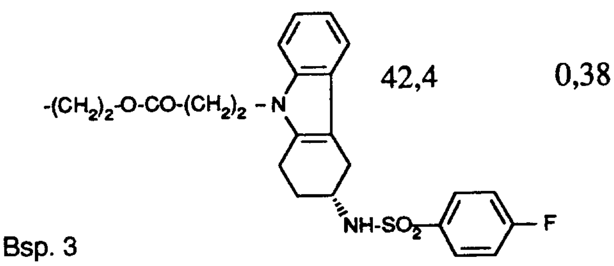 | 42,4 | 0,38 (f) |

*2 getrennte Diastereomere von Bsp. 3

Fortsetzung Tabelle 1:

| Bsp.-Nr. | Y | $R^3$ | Ausbeute (% d.Th.) / $R_f$ (LM) | |
|---|---|---|---|---|
| 9 | m-$NO_2$ | $-C(CH_3)_3$ | 50,6 | 0,55 (c) |
| 10 | m-$NO_2$ | $-(CH_2)_2-OCH_3$ | 55,8 | 0,51 (c) |
| 11 | m-$NO_2$ | $-CH_2-C_6H_5$ | 32,3 | 0,59 (c) |
| 12 | m-$NO_2$ | -n-$C_4H_9$ | 43,9 | 0,61 (c) |
| 13 | o-$NO_2$ | -n-$C_4H_9$ | 36,0 | 0,57 (c) |
| 14 | p-$NO_2$ | $- CH(CH_3)_2$ | 78,1 | 0,35 (g) |
| 15 | o-Cl | $-C_2H_5$ | 51,6 | 0,45 (c) |
| 16 | o-Cl | $-CH(CH_3)_2$ | 85,2 | 0,3 (h) |
| 17 | o-Cl | -n-$C_4H_9$ | 72,3 | 0,63 (c) |
| 18 | m-Cl | $-CH_3$ | 81 | 0,25 (h) |
| 19 | m-Cl | $-CH(CH_3)_2$ | 75,8 | 0,31 (h) |
| 20 | m-Cl | $-C_2H_5$ | 56,3 | 0,34 (c) |
| 21 | m-Cl | -n-$C_4H_9$ | 47,7 | 0,51 (c) |
| 22 | H | $-CH(CH_3)_2$ | 60 | 0,55 (c) |
| 23 | o-$CF_3$ | -n-$C_4H_9$ | 44,3 | 0,62 (c) |

Tabelle 2:

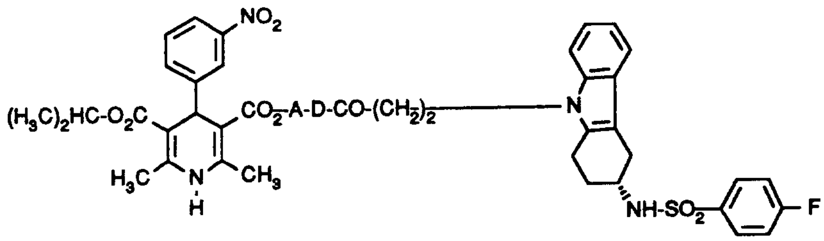

| Bsp.-Nr. | A-D | Ausbeute (% d.Th.) / $R_f$ (LM) | |
|---|---|---|---|
| 24 | 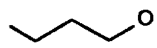 | 67,9 | 0,37 (g) |
| 25 | 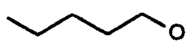 | 69,4 | 0,41 (g) |
| 26 | 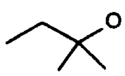 | 74,3 | 0,57 (g) |
| 27 | 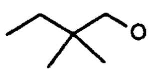 | 61,1 | 0,55 (g) |

Tabelle 3:

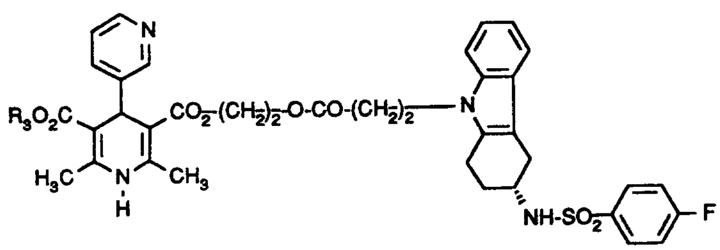

| Bsp.-Nr. | $R^3$ | Ausbeute (% d.Th.) / $R_f$ (LM) | |
|---|---|---|---|
| 28 | $-C_2H_5$ | 46,6% | 0,35 (d) |

Methode C:

Allgemeine Arbeitsvorschrift zur Herstellung der Verbindungen der allgemeinen Formel (I), in welcher D für NH steht, durch Veresterung verschiedener DHP-Halbester der allgemeinen Formel (II) mit (3R)-3-(4-Fluorphenylsulfonamido)-1,2,3,4-tetrahydrocarbazol-9-propansäureethanolamid (Verbindung aus Beispiel IV).

7 mmol DHP-Halbester und 3,22 g (7 mmol) der Verbindung aus Beispiel IV werden in 20 ml abs. THF gelöst. Bei 0°C werden 100 mg DMAP und 1,87 g (9,1 mmol) Dicyclohexylcarbodiimid zugegeben. Es wird 30 min bei 0°C, dann 3 h bei 20°C gerührt. Der Niederschlag wird abfiltriert, das THF abdestilliert und der Rückstand in 50 ml Essigsäureethylester aufgenommen. Es wird 2 mal mit gesättigter aq. $Na_2CO_3$-Lösung, 1 mal mit 1 n HCl und 1 x mit gesättiger NaCl-Lösung ausgeschüttelt, die organische Phase über $Na_2SO_4$ getrocknet und eingedampft. Der Rückstand wird durch Chromatographie gereinigt (Eluens: Toluol / Aceton-Gemische 25:1 bis 5:1).

Methode D:

Allgemeine Arbeitsvorschrift zur Herstellung der Verbindungen der allgemeinen Formel I, in welcher D für NH steht, durch Umsetzung von Verbindungen der allgemeinen Formel (IV:X = $NH_2$) mit (3R)-3-(4-Fluorphenylsulfonamido)-1,2,3,4-tetrahydrocarbazol-9-propancarbonsäure.

4,16 g (10 mmol) (3R)-3-(4-Fluorphenylsulfonamido)-1,2,3,4-tetrahydrocarbazol-9-propancarbonsäure, 1,35 g (10 mmol) N-Hydroxy-benzotriazol und 2,27 g (11 mmol) Dicyclohexylcarbodiimid in 30 ml abs. THF werden 1 h bei 0°C und 1 h bei Raumtemperatur gerührt. Nach Zugabe von 10 mmol eines Amins der allgemeinen Formel (IV:X = $NH_2$) wird noch 3 h bei Raumtemperatur gerührt, abfiltriert und eingedampft. Der Rückstand wird in 50 ml $CH_2Cl_2$ gelöst, mit 1 n HCl-Lösung, 1 n NaOH-Lösung und ges. NaCl-Lösung gewaschen, über $Na_2SO_4$ getrocknet und eingedampft. Der Rückstand wird über Kieselgel mit Toluol/Aceton 5:1 chromatographiert.

Die in Tabelle 4 aufgeführten Beispiele werden nach den oben aufgeführten allgemeinen Herstellungsvorschriften hergestellt:

Tabelle 4:

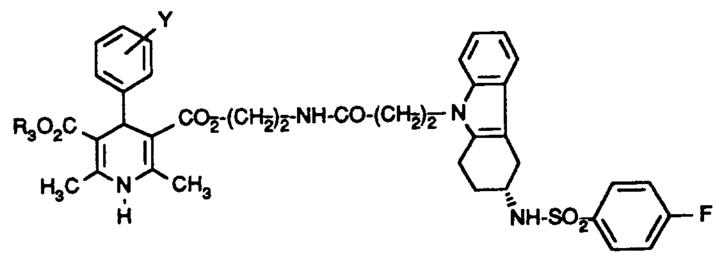

| Bsp.-Nr. | Y | $R^3$ | Ausbeute (% d.Th.) / $R_f$ (LM) | |
|---|---|---|---|---|
| 29 | m-$NO_2$ | -$CH(CH_3)_2$ | 79,1 | 0,46 (h) |
| 30 | m-Cl | -$CH(CH_3)_2$ | 75 | 0,36 (k) |
| 31 | o-$CF_3$ | -$CH(CH_3)_2$ | 54,6 | 0,57 (k) |
| 32 | m-$NO_2$ | -$CH_3$ | 72,3 | 0,34(k) |
| 33 | m-Cl | -$CH_3$ | 67,5 | 0,29(k) |
| 34 | o-Cl | -$CH_3$ | 66,1 | 0,27(k) |
| 35 | o,m-$Cl_2$ | -$CH_3$ | 73,8 | 0,37(k) |

**Patentansprüche**

**1.** Indolsulfonamid substituierte Dihydropyridine der allgemeinen Formel

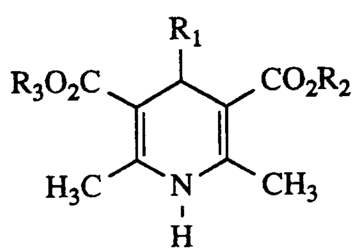

(I),

in welcher

$R^1$ für Aryl mit 6 bis 10 Kohlenstoffatomen oder für einen 5- bis 7-gliedrigen ungesättigten Heterocyclus mit bis zu 2 Heteroatomen aus der Reihe S, N oder O steht, die gegebenenfalls bis zu 3-fach gleich oder verschieden durch Halogen, Nitro, Trifluormethyl, Trifluormethoxy, Cyano, Difluormethoxy, geradkettiges oder

verzweigtes Alkyl, Alkoxy oder Alkylthio mit jeweils bis zu 8 Kohlenstoffatomen, Benzyl oder Phenoxy substituiert sind,

$R^2$ für einen Rest der Formel

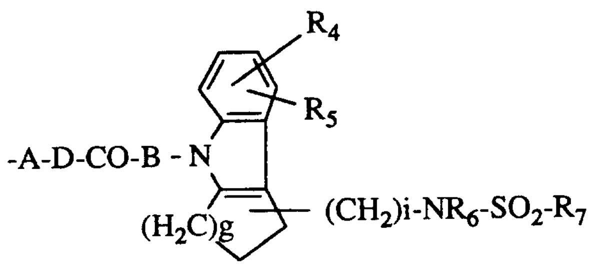

steht,

worin

A und B gleich oder verschieden sind und eine Gruppe der Formel

$-(CH_2)_b-(CR^8R^9)_d-(CH_2)_e$ bedeuten,

worin

b eine Zahl 1, 2, 3, 4 oder 5 bedeutet,

d und e gleich oder verschieden sind und eine Zahl 0, 1, 2, 3, 4 oder 5 bedeuten,

$R^8$ und $R^9$ gleich oder verschieden sind und Wasserstoff oder geradkettiges oder verzweigtes Alkyl mit bis zu 6 Kohlenstoffatomen bedeuten,

D ein Sauerstoffatom oder die -NH-Gruppe bedeutet,

g eine Zahl 1 oder 2 bedeutet,

i eine Zahl 0, 1, 2 oder 3 bedeutet,

$R^4$ und $R^5$ gleich oder verschieden sind und Wasserstoff, Aryl mit 6 bis 10 Kohlenstoffatomen, Nitro, Cyano, Halogen, Trifluormethyl, Trifluormethoxy, Carboxy, Hydroxy oder geradkettiges oder verzweigtes Alkyl, Alkoxy oder Alkoxycarbonyl mit jeweils bis zu 6 Kohlenstoffatomen bedeuten,

$R^6$ Wasserstoff, geradkettiges oder verzweigtes Alkyl mit bis zu 8 Kohlenstoffatomen oder Phenyl bedeutet,

$R^7$ Aryl mit 6 bis 10 Kohlenstoffatomen oder einen 5- bis 7-gliedrigen ungesättigten Heterocyclus mit bis zu 2 Heteroatomen aus der Reihe S, N oder O bedeutet, die gegebenenfalls bis zu 3-fach gleich oder verschieden durch Halogen, Cyano, Nitro, Trifluormethyl, Trifluormethoxy, Trifluormethylthio, Hydroxy, Carboxy, Phenyl, Phenoxy, Benzyloxy, Benzylthio, geradkettiges oder verzweigtes Alkoxy, Alkyl, Carboxyalkyl, Alkoxycarbonyl oder Alkoxycarbonylalkyl mit jeweils bis zu 8 Kohlenstoffatomen oder durch eine Gruppe der Formel $-NR^{10}R^{11}$ substituiert sind,

worin

$R^{10}$ und $R^{11}$ gleich oder verschieden sind und Wasserstoff, geradkettiges oder verzweigtes Alkyl mit bis zu 6 Kohlenstoffatomen, Phenyl oder Benzyl bedeuten,

$R^3$ für geradkettiges oder verzweigtes Alkyl oder Alkenyl mit jeweils bis zu 10 Kohlenstoffatomen steht, die gegebenenfalls bis zu 2-fach gleich oder verschieden durch Carboxy, geradkettiges oder verzweigtes Alkylthio, Alkoxy, Alkoxycarbonyl, Acyl oder Acyloxy mit jeweils bis zu 8 Kohlenstoffatomen, Phenyl, Phenoxy, Carboxy, Hydroxy oder durch die Gruppe $-NR^{10}R^{11}$ substituiert sind,

worin

$R^{10}$ und $R^{11}$ die oben angegebene Bedeutung haben,

oder für die Gruppe der Formel

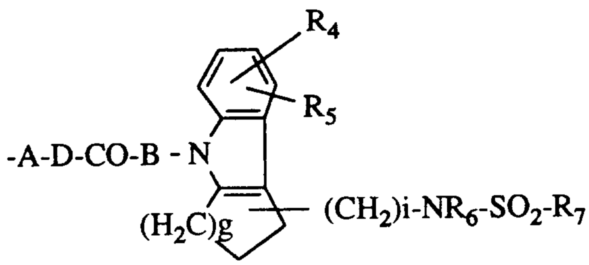

steht,

worin

A, B, D, g, i, $R^4$, $R^5$, $R^6$ und $R^7$ die oben angegebene Bedeutung haben,

gegebenenfalls in einer isomeren Form und deren Salze.

**2.** Indolsulfonamid substituierte Dihydropyridine nach Anspruch 1, wobei

$R^1$ für Phenyl, Naphthyl, o-Pyridyl, m-Pyridyl, p-Pyridyl oder Thienyl steht, die gegebenenfalls bis zu 2-fach gleich oder verschieden durch Fluor, Chlor, Brom, Nitro, Trifluormethyl, Trifluormethoxy, geradkettiges oder verzweigtes Alkyl oder Alkoxy mit jeweils bis zu 6 Kohlenstoffatomen, Benzyl oder Phenoxy substituiert sind,

$R^2$ für einen Rest der Formel

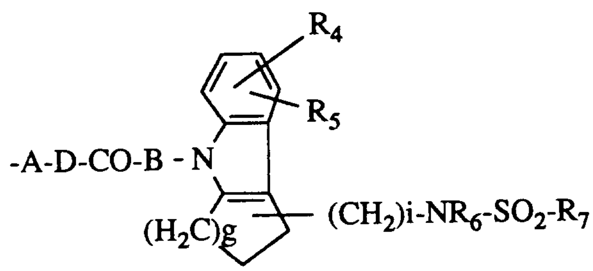

steht,

worin

A und B gleich oder verschieden sind und eine Gruppe der Formel

$-(CH_2)_b-(CR^8R^9)_d-(CH_2)_e$ bedeuten,

worin

b eine Zahl 1, 2, 3 oder 4 bedeutet,

d und e gleich oder verschieden sind und eine Zahl 0, 1, 2 oder 3 bedeuten,

$R^8$ und $R^9$ gleich oder verschieden sind und Wasserstoff, geradkettiges oder verzweigtes Alkyl mit bis zu 4 Kohlenstoffatomen bedeuten,

D ein Sauerstoffatom oder die -NH-Gruppe bedeutet,

g eine Zahl 1 oder 2 bedeutet,

i eine Zahl 0, 1 oder 2 bedeutet,

$R^4$ und $R^5$ gleich oder verschieden sind und Wasserstoff, Phenyl, Nitro, Cyano, Fluor, Chlor, Brom, Trifluormethyl oder geradkettiges oder

verzweigtes Alkyl oder Alkoxy mit jeweils bis zu 4 Kohlenstoffatomen bedeuten,

$R^6$ Wasserstoff, geradkettiges oder verzweigtes Alkyl mit bis zu 6 Kohlenstoffatomen bedeutet,

$R^7$ Phenyl, Naphthyl oder Thienyl bedeutet, die gegebenenfalls bis zu 2-fach gleich oder verschieden durch Fluor, Chlor, Trifluormethyl, Trifluormethoxy, Hydroxy, Carboxy oder durch geradkettiges oder verzweigtes Alkoxy, Alkyl oder Alkoxycarbonyl mit jeweils bis zu 6 Kohlenstoffatomen oder durch eine Gruppe der Formel $-NR^{10}R^{11}$ substituiert sind,

worin

$R^{10}$ und $R^{11}$ gleich oder verschieden sind und Wasserstoff, geradkettiges oder verzweigtes Alkyl mit bis zu 4 Kohlenstoffatomen, Phenyl oder Benzyl bedeuten,

$R^3$ für geradkettiges oder verzweigtes Alkyl mit bis zu 8 Kohlenstoffatomen steht, das gegebenenfalls durch geradkettiges oder verzweigtes Alkoxy, Alkylthio, Alkoxycarbonyl, Acyl oder Acyloxy mit jeweils bis zu 6 Kohlenstoffatomen, Phenyl, Phenoxy, Carboxy oder Hydroxy oder durch die Gruppe $-NR^{10}R^{11}$ substituiert ist,

worin

$R^{10}$ und $R^{11}$ die oben angegebene Bedeutung haben,

oder für die Gruppe der Formel

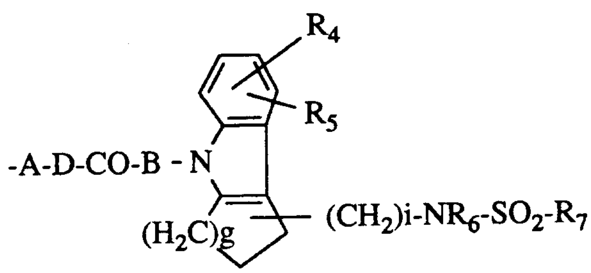

steht,

worin

A, B, D, g, i, $R^4$, $R^5$, $R^6$ und $R^7$ die oben angegebene Bedeutung haben,

gegebenenfalls in einer isomeren Form und deren Salze.

**3.** Indolsulfonamid substituierte Dihydropyridine nach Anspruch 1, wobei

$R^1$ für Phenyl, Naphthyl, m-Pyridyl oder Thienyl steht, die gegebenenfalls bis zu zweifach gleich oder verschieden durch Fluor, Chlor, Nitro, Trifluormethyl, Trifluormethoxy, geradkettiges oder verzweigtes Alkyl oder Alkoxy mit jeweils bis zu 4 Kohlenstoffatomen, Benzyl oder Phenoxy substituiert sind,

$R^2$ für einen Rest der Formel

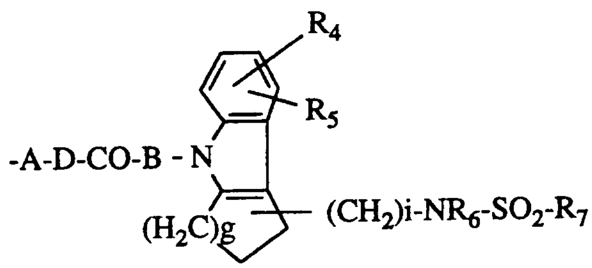

steht,

worin

A und B gleich oder verschieden sind und eine Gruppe der Formel

$-(CH_2)_b-(CR^8R^9)_d-(CH_2)_e$ bedeuten,

worin

b eine Zahl 1, 2 oder 3 bedeutet,

d und e gleich oder verschieden sind und eine Zahl 0, 1 oder 2 bedeuten,

$R^8$ und $R^9$ gleich oder verschieden sind und Wasserstoff, Methyl oder Ethyl bedeuten,

D ein Sauerstoffatom oder die -NH-Gruppe bedeutet,

g eine Zahl 1 oder 2 bedeutet,

i eine Zahl 0 oder 1 bedeutet,

$R^4$ und $R^5$ gleich oder verschieden sind und Wasserstoff, Phenyl, Nitro, Fluor, Chlor, Trifluormethyl, Methyl oder Methoxy bedeuten,

$R^6$ Wasserstoff, geradkettiges oder verzweigtes Alkyl mit bis zu 4 Kohlenstoffatomen bedeutet,

$R^7$ Phenyl, Naphthyl oder Thienyl bedeutet, die gegebenenfalls durch Fluor, Chlor, Trifluormethyl, Trifluormethoxy oder durch geradkettiges oder verzweigtes Alkoxy oder Alkyl mit jeweils bis zu 4 Kohlenstoffatomen substituiert sind,

$R^3$ für geradkettiges oder verzweigtes Alkyl mit bis zu 6 Kohlenstoffatomen steht, das gegebenenfalls durch geradkettiges oder verzweigtes Alkoxy, Alkylthio, Alkoxycarbonyl oder Acyl mit jeweils bis zu 4 Kohlenstoffatomen, Phenyl oder Phenoxy substituiert ist, oder für die Gruppe der Formel

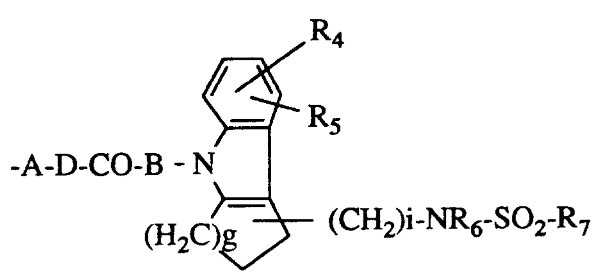

steht,

worin

A, B, D, g, i, $R^4$, $R^5$, $R^6$ und $R^7$ die oben angegebene Bedeutung haben,

gegebenenfalls in einer isomeren Form und deren Salze.

**4.** Indolsulfonamid substituierte Dihydropyridine nach Anspruch 1, wobei

$R^1$ für Phenyl oder Pyridyl steht, die gegebenenfalls durch Fluor, Chlor, Nitro oder Trifluormethyl substituiert sind,

$R^2$ für einen Rest der Formel

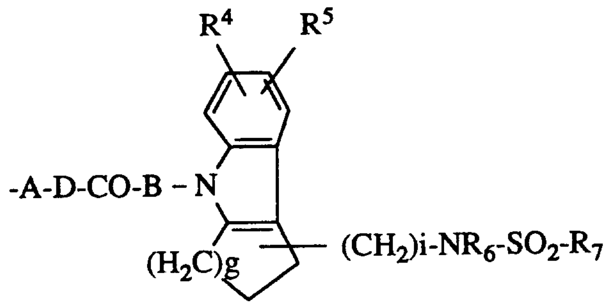

steht,

worin

A - eine Gruppe der Formel $-(CH_2)_b - (CR^8R^9)_d - (CH_2)_e$ bedeutet,

wobei

b - für eine Zahl 1, 2 oder 3 steht,

d, e - gleich oder verschieden sind und für eine Zahl 0, 1 oder 2 stehen

$R^8$, $R^9$ - gleich oder verschieden sind und für Wasserstoff oder Methyl stehen

D - ein Sauerstoffatom oder eine NH-Gruppe bedeutet

g - die Zahl 2 bedeutet

i - die Zahl 0 bedeutet

$R^4$ und $R^5$ - Wasserstoff bedeuten

$R^6$ - Wasserstoff bedeutet

$R^7$ - Phenyl bedeutet, das gegebenenfalls durch Fluor oder Chlor substituiert ist,

$R^3$ für geradkettiges oder verzweigtes Alkyl mit bis zu 6 Kohlenstoffatomen steht, oder für eine Gruppe der Formel

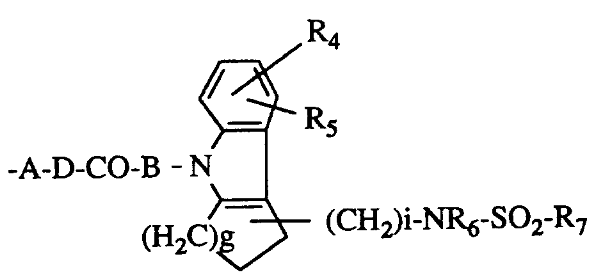

steht,

worin

A, B, D, g, i, $R^4$, $R^5$, $R^6$ und $R^7$ die oben angegebene Bedeutung haben

gegebenenfalls in einer isomeren Form und deren Salze.

**5.** Indolsulfonamid substituierte Dihydropyridine nach Anspruch 1 zur Bekämpfung von Krankheiten.

**6.** Verfahren zur Herstellung von Indolsulfonamid substituierten Dihydropyridinen der Formel

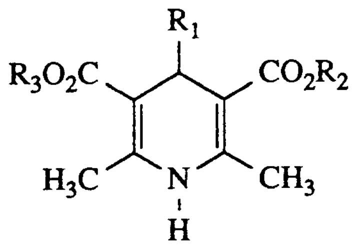

(I),

in welcher

$R^1$ für Aryl mit 6 bis 10 Kohlenstoffatomen oder für einen 5- bis 7-gliedrigen ungesättigten Heterocyclus mit bis zu 2 Heteroatomen aus der Reihe S, H oder O steht, die gegebenenfalls bis zu 3-fach gleich oder verschieden durch Halogen, Nitro, Trifluormethyl, Trifluormethoxy, Cyano, Difluormethoxy, geradkettiges oder verzweigtes Alkyl, Alkoxy oder Alkylthio mit jeweils bis zu 8 Kohlenstoffatomen, Benzyl oder Phenoxy substituiert sind,

$R^2$ für einen Rest der Formel

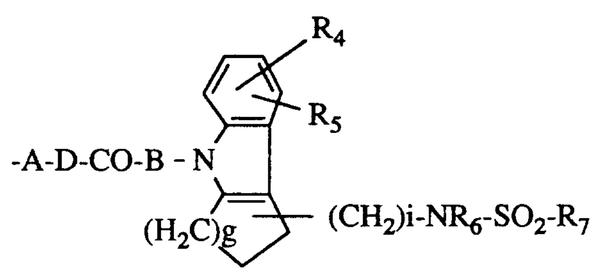

steht,

worin

A und B gleich oder verschieden sind und eine Gruppe der Formel

$-(CH_2)_b-(CR^8R^9)_d-(CH_2)_e$ bedeuten,

worin

b eine Zahl 1, 2, 3, 4 oder 5 bedeutet,

d und e gleich oder verschieden sind und eine Zahl 0, 1, 2, 3, 4 oder 5 bedeuten,

$R^8$ und $R^9$ gleich oder verschieden sind und Wasserstoff oder geradkettiges oder verzweigtes Alkyl mit bis zu 6 Kohlenstoffatomen bedeuten,

D ein Sauerstoffatom oder die -NH-Gruppe bedeutet,

g eine Zahl 1 oder 2 bedeutet,

i eine Zahl 0, 1, 2 oder 3 bedeutet,

$R^4$ und $R^5$ gleich oder verschieden sind und Wasserstoff, Aryl mit 6 bis 10 Kohlenstoffatomen, Nitro, Cyano, Halogen, Trifluormethyl, Trifluormethoxy, Carboxy, Hydroxy oder geradkettiges oder verzweigtes Alkyl, Alkoxy oder Alkoxycarbonyl mit jeweils bis zu 6 Kohlenstoffatomen bedeuten,

$R^6$ Wasserstoff, geradkettiges oder verzweigtes Alkyl mit bis zu 8 Kohlenstoffatomen oder Phenyl bedeutet,

$R^7$ Aryl mit 6 bis 10 Kohlenstoffatomen oder einen 5- bis 7-gliedrigen ungesättigten Heterocyclus mit bis zu 2 Heteroatomen aus der Reihe S, N oder O bedeutet, die gegebenenfalls bis zu 3-fach gleich oder verschieden durch Halogen, Cyano, Nitro, Trifluormethyl, Trifluormethoxy, Trifluormethylthio, Hydroxy, Carboxy, Phenyl, Phenoxy, Benzyloxy, Benzylthio, geradkettiges oder verzweigtes Alkoxy, Alkyl, Carboxyalkyl, Alkoxycarbonyl oder Alkoxycarbonylalkyl mit jeweils bis zu 8 Kohlenstoffatomen oder durch eine Gruppe der Formel $-NR^{10}R^{11}$ substituiert sind,

worin

$R^{10}$ und $R^{11}$ gleich oder verschieden sind und Wasserstoff, geradkettiges oder verzweigtes Alkyl mit bis zu 6 Kohlenstoffatomen, Phenyl oder Benzyl bedeuten,

$R^3$ für geradkettiges oder verzweigtes Alkyl oder Alkenyl mit jeweils bis zu 10 Kohlenstoffatomen steht, die gegebenenfalls bis zu 2-fach gleich oder verschieden durch Carboxy, geradkettiges oder verzweigtes Alkylthio, Alkoxy, Alkoxycarbonyl, Acyl oder Acyloxy mit jeweils bis zu 8 Kohlenstoffatomen, Phenyl, Phenoxy, Carboxy, Hydroxy oder durch die Gruppe $-NR^{10}R^{11}$ substituiert sind,

worin

$R^{10}$ und $R^{11}$ die oben angegebene Bedeutung haben,

oder für die Gruppe der Formel

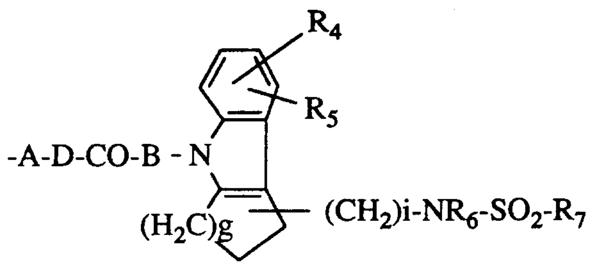

steht,

worin

A, B, D, g, i, $R^4$, $R^5$, $R^6$ und $R^7$ die oben angegebene Bedeutung haben,

gegebenenfalls in einer isomeren Form und deren Salze,

dadurch gekennzeichnet, daß man

[A] Dihydropyridincarbonsäuren der allgemeinen Formel (II)

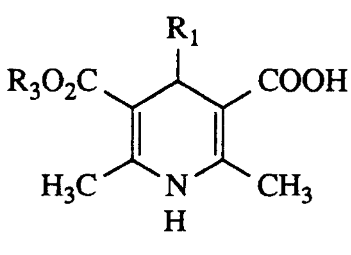

(II).

in welcher

$R^1$ und $R^3$ die oben angegebene Bedeutung haben

mit Indolsulfonamiden der allgemeinen Formel (III)

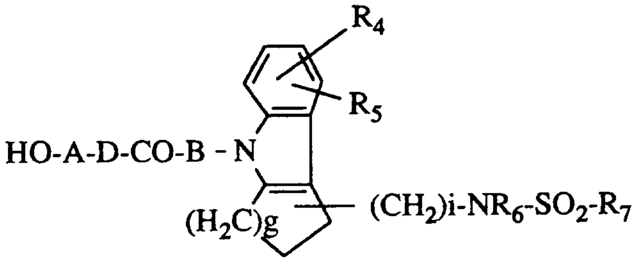

(III),

in welcher

A, D, B, g, i, $R^4$, $R^5$, $R^6$ und $R^7$ die oben angegebene Bedeutung haben,

in inerten Lösemitteln, gegebenenfalls in Anwesenheit einer Base und/oder eines Hilfsstoffes, und gegebenenfalls unter Aktivierung der Carbonsäure verestert,

oder daß man

[B] Dihydropyridine der allgemeinen Formel (IV)

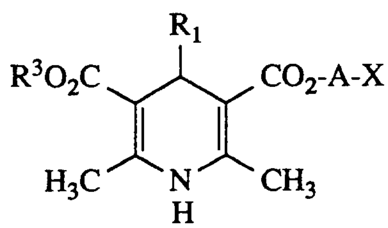

(IV),

worin

$R^1$, $R^3$ und A die oben angegebene Bedeutung haben und

X entweder für eine -OH-Gruppe steht, wenn D ein Sauerstoffatom bedeutet, oder für eine $-NH_2$-Gruppe steht, wenn D eine -NH-Gruppe bedeutet,

mit Indolsulfonamidsäuren der allgemeinen Formel (V),

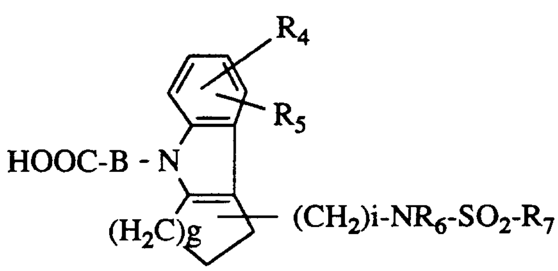

(V),

in welcher

B, g, i, $R^4$, $R^5$, $R^6$ und $R^7$ die oben angegebene Bedeutung haben,

in inerten Lösemitteln, gegebenenfalls in Anwesenheit eines Hilfstoffes und gegebenenfalls unter Aktivierung der Carbonsäure kondensiert,

und im Fall der reinen Enantiomeren zunächst auf der Stufe der Säuren der allgemeinen Formel (II) eine Enantiomerentrennung nach üblicher Methode durchführt und die entsprechenden enantiomerenreinen Säuren mit den ebenfalls nach einer Diastereomerentrennung im Verlauf der Synthese anfallenden enantiomerenreinen Verbindungen der allgemeinen Formel (III) oder nach Überführung der enantiomerenreinen Säuren (II) in die enantiomerenreinen Verbindungen (IV) mit den enantiomerenreinen Verbindungen der allgemeinen Formel (V) umsetzt.

**7.** Arzneimittel enthaltend mindestens ein Indolsulfonamid substituiertes Dihydropyridin nach Anspruch 1.

**8.** Arzneimittel nach Anspruch 7 zur Behandlung von Herz-, Kreislauf- und thromboembolischen Erkrankungen.

**9.** Verwendung von Indolsulfonamid substituierten Dihydropyridinen nach Anspruch 1 zur Herstellung von Arzneimitteln.

**10.** Verwendung von Indolsulfonamid substituierten Dihydropyridinen nach Anspruch 9 zur Herstellung von Arzneimitteln zur Bekämpfung von Herz-, Kreislauf- und thromboembolischen Erkrankungen.

Europäisches Patentamt

# EUROPÄISCHER RECHERCHENBERICHT

Nummer der Anmeldung

EP 92 11 5274

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | EP-A-0 242 518 (BAYER)<br>28. Oktober 1987<br>* das ganze Dokument *<br>& DE-A-3 631 824 (BAYER)<br>31. März 1988 | 1-10 | C07D401/12<br>C07D401/14<br>A61K31/445 |
| D,A | EP-A-0 265 947 (GREEN CROSS)<br>5. April 1988<br>* das ganze Dokument * | 1-10 | |
| A | EP-A-0 247 345 (BRISTOL-MYERS)<br>2. Dezember 1987<br>* Beispiel 33 *<br>* Anspruch 1 * | 1-10 | |

RECHERCHIERTE SACHGEBIETE (Int. Cl.5)

C07D
A61K

Der vorliegende Recherchenbericht wurde für alle Patentansprüche erstellt

| Recherchenort | Abschlußdatum der Recherche | Prüfer |
|---|---|---|
| DEN HAAG | 13 NOVEMBER 1992 | Bernd Kissler |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument